(19) Europäisches Patentamt
European Patent Office
Office européen des brevets

(11) Publication number: **0 045 571**
**B1**

(12) # EUROPEAN PATENT SPECIFICATION

(45) Date of publication of patent specification: **26.06.85**

(51) Int. Cl.$^4$: **C 07 C 47/228,** C 07 C 45/00, C 07 C 33/20, C 07 C 29/32

(21) Application number: **81303124.2**

(22) Date of filing: **08.07.81**

(54) **Process for the production of phenyl substituted aldehydes and of phenyl substituted alcohols.**

(30) Priority: **09.07.80 GB 8022381**
**31.10.80 US 202608**

(43) Date of publication of application:
**10.02.82 Bulletin 82/06**

(45) Publication of the grant of the patent:
**26.06.85 Bulletin 85/26**

(84) Designated Contracting States:
**AT BE CH DE FR GB IT LI NL SE**

(56) References cited:
**EP-A-0 032 576**
**US-A-3 925 458**

**G.A. OLAH "Friedl-Crafts and related reactions", vol. II, part 1, "Alkylation and related reactions" 1964, INTERSCIENCE PUBLISHERS, New York-London-Sydney, pages 21-27, 417-424, 428, 514-524**
**HOUBEN-WEYL "Methoden der organischen Chemie", 4th edition, vol. VII, "Sauerstoffverbindungen", part 1, Aldehyde, 1954, GEORG THIEME VERLAG, Stuttgart, pages 164-166**

(73) Proprietor: **BUSH BOAKE ALLEN Limited**
**Blackhorse Lane Walthamstow**
**London E17 5QP (GB)**

(72) Inventor: **Webb, David**
**27, Edwards Way**
**Hutton Essex CM13 1BT (GB)**

(74) Representative: **Perry, Robert Edward et al**
**GILL JENNINGS & EVERY 53-64 Chancery Lane**
**London WC2A 1HN (GB)**

**EP 0 045 571 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The preparation and odiferous properties of a number of phenyl substituted propanals of the general formula:—

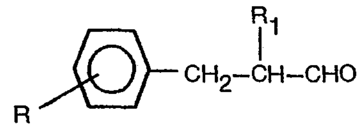

wherein R represents an alkyl group having from 1 to 5 carbon atoms and $R^1$ represents a hydrogen atom or a methyl group has been reviewed by Berends and van der Linde. (Perfumery and Essential Oil Record June 1967 p 372—378). Of the aldehydes which they prepared those wherein R represents an isopropyl or a tertiary butyl group have become articles of commerce by virtue of their utility as ingredients of compounded perfumery compositions. The compound were R represent a para tertiary butyl group and $R^1$ represents a methyl group is particularly valuable in this respect. These aldehydes are synthetic organic chemicals which have not been found in nature. Because of their known value the industry has devoted a good deal of effort to devising synthetic methods whereby these aldehydes can be manufactured more cheaply and efficiently. Besides those methods which are reviewed by Berends and van der Linde a number of other synthetic techniques have recently been proposed see for example USP 4070374 and British Patent Application 2009151. Methods of substituting alkyl groups into aromatic rings are reviewed in "Friedel-Crafts and Related Reactions" (1964) ed. G. A. Olah Vol. II part I (Interscience Publishers) pp. 24 *et seq.*, 417 *et seq* and 515 *et seq*). Processes involving oxidation of corresponding alcohols have been discussed in "MeHoden der Organishen Chemie" (Houben-Weyl) 4th ed. (1954) ed. E. Mueller, Vol VII, part I, "Sauerstoff-Verbindungen II" Part I "Aldehyde" pp. 164—166.

We have now discovered a novel process whereby certain of the aldehydes having the general formula described above can be prepared in high yield and at lost cost. From one aspect our invention provides a process for the manufacture of an aldehyde having the formula I.

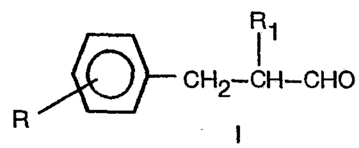

I

wherein R represents a secondary or tertiary alkyl group having 3 or 4 carbon atoms and $R^1$ represents a hydrogen atom or a methyl group which comprises reducing an aldehyde which is 3-phenyl prop-2-enal or 3 phenyl 2-methyl prop-2-enal to the corresponding 3 phenyl propanol, alkylating said phenyl propanol with an appropriate alkylating agent so as to produce a product comprising a 3-(mono alkyl phenyl) propanol wherein the alkyl group is a secondary or tertiary alkyl group comprising 3 or 4 carbon atoms and oxidising said 3-(mono alkyl phenyl) propanol so as to produce the corresponding propanal.

In a preferred embodiment the invention provides a process for the manufacture of a compound having the formula 1, wherein the substituent R is in the para position on the aromatic ring.

The aldehyde starting material may conveniently be produced by the condensation of benzaldehyde and propionaldehyde or acetaldehyde which is an example of an aldol condensation and can be carried out using any of the reaction techniques which are known *per se* to be useful for this reaction. By the term "reaction techniques known *per se*" in the specification is meant techniques for the reaction which are or have been used or described in the chemical literature. Thus the propionaldehyde or acetaldehyde may be added to a stirred solution of benzaldehyde in a suitable solvent e.g. an aqeuous solution of an aliphatic alcohol such as methanol in the presence of a suitable basic catalyst e.g. sodium hydroxide. The reactants are preferably maintained at a temperature in the range 20 to 80°C for a period of up to 3 hours. The reaction is normally carried out using at least a molar excess of benzaldehyde so as to minimise by-product formation. The reaction products may be separated using conventional techniques, e.g. fractional distillation. The separation of the 3-phenyl prop-2-enal is a strongly preferred. The preferred aldehyde for present use is propionaldehyde the preferred products being those wherein $R_1$ represents a methyl group.

The product aldehyde may be reduced to the corresponding alcohol using the reaction techniques for such a type of reduction known *per se*. Conveniently, the reduction can be carried out by the catalytic hydrogenation of the aldehyde. This hydrogenation may be effected in the presence of a suitable catalyst, e.g. copper chromite at a temperature of from 120 to 180°C and a pressure of 100 to 150 p sig. hydrogen. Once the uptake of hydrogen has ceased the reaction mixture is filtered and the desired alcohol separated by distillation.

The second stage of the process produces a 3-(alkyl phenyl)-propanol ie one having the formula:

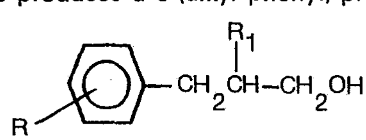

wherein R and $R^1$ are as defined above, which process comprises alkylating a 3-phenyl propanol with an alkylating agent so as to produce a product comprising said 3-(alkyl phenyl) propanol. The described multistage process of the invention is described also in European application 32576 but that application does not describe the preferred way of performing the second stage, namely alkylation in the presence of protonic acid catalyst using alkylating agents selected from alkanols (generally secondary or tertiary alkanols) and alkenes having 3 or 4 carbon atoms or oligomers of such alkenes. The invention also includes, as a separate aspect, this process conducted in the presence of concentrated phosphoric acid.

Instead of using a protonic acid catalyst the alkylation may be alkylation with alkylating agents which are secondary or tertiary alkyl halides having 3 or 4 carbon atoms and catalysts which are Lewis acids such as aluminium chloride, ferric chloride and zinc chloride.

The product of the alkylation may comprise a mixture of dialkylated and monoalkylated species and isomers thereof together with any unreacted starting materials. The relative proportions of these various possible products which are formed varies with the nature of the reactants, and catalyst and according to the conditions under which the alkylation is carried out. We have found that it is possible to influence the proportion of the various possible alkylated products by specific choice of the reaction conditions.

When alkylating with Lewis Acid catalyst the choice and volume of the solvent, the mode of addition of the reactants and catalyst, the molar ratio of the reactants and their concentration can all exert an appreciable effect upon the composition of the product although in general less than that exerted by the nature of reactants and the particular catalyst which is used. Nevertheless, the particular conditions under which the reaction is carried out may well make a significant difference to the composition of the desired product.

The preferred alkyl halide alkylating agents for present use are chlorides especially tertiary butyl chloride and isopropyl chloride. The preferred Lewis acid catalysts are aluminium chloride and ferric chloride.

Using this alkylating system the reaction is preferably carried out by mixing the halide and the primary alcohol and adding the resulting mixture to a solution or suspension of the catalyst. The solvent may be any of those known for use in Friedel Crafts alkylations, e.g. nitrobenzene or carbon disulphide. Preferably ethylene dichloride or di-chloromethane is employed as the solvent. The temperature of the reactants is preferably maintained within the range −30° to 40°C. The utilisation of lower temperatures appears to increase the proportion of para to meta alkylated product, but in general the lower the temperature the lower degree of conversion of the starting material, so in the preferred embodiment of this invention where the aim of the alkylation is the production of the para substituted product the temperature employed will be selected so as to achieve a compromise.

The phenyl propanol and the alkyl chloride will normally be employed in approximately equimolar quantities. Larger proportions of phenyl propanol will favour the selective formation of a para substituted products but the use of such excesses is less preferred because of the consequent lower degree of conversion. The Lewis acid catalysts may be employed in any convenient quantity but in general we prefer to use from 0.3 to 1.0 moles most preferably 0.4 to 0.6 moles of catalyst per mole of phenyl propanol. Preferably a volume of solvent which is from 1 to 6 times the total volume of the reactants is employed. The use of a larger volume of solvent appears to favour the selective formation of a para substituted product but this is in general uneconomic and we prefer to employ a volume of solvent which is from 2 to 5 times the volume of the reactants.

The product mixture may be allowed to warm to ambient temperature if appropriate, at the end of the reaction and is then washed and dried.

The product mixture can be separated from any unchanged starting material using conventional techniques e.g. fractional distillation.

The particular technique for the alkylating procedures of this invention using protonic acid catalysts varies with the particular nature of the alkylating agent. The preferred alkenes for use according to our invention are propylene and isobutylene which are gases at the temperatures at which the processes will be carried out. The preferred technique for operating an alkylating processs involving the use of gaseous alkene is to bubble the gas through a liquid medium comprising the other reactants.

The alcohols useful to our invention are in essence those which are dehydrated and protonated in situ to generate an appropriate carbonium ion. Preferred alcohols for present use are isopropanol, and tertiary butanol. These alcohols are preferably added in small increments to the reaction medium as the reaction progresses.

The preferred oligomeric alkenes for present use are di-isobutylene (a mixture of 2,4,4 trimethyl pent-1-ene and 2,4,4 trimethyl pent-2-ene) and tri-isobutylene (a mixture of dodecanes consisting predominantly of 2,4,4,6,6 pentamethyl-hept-1-ene). Alkylation processes using these oligomers as the alkylating agents are preferably carried out at a temperature below the boiling point of the oligomer so that the liquid oligomer can be gradually added to the other reactants during the alkylation reaction.

Any acid which is sufficiently strong to protonate the alkylating agent and thereby generate an appropriate carbonium ion can be used to catalyse the reaction. However, acids which react chemically and irreversibly with the primary phenyl substituted alcohol should not be employed. The preferred catalyst for present use is phosphoric acid. The use of a concentrated acid i.e. at least 85% and preferably at least 90% acid being especially preferred.

The alkylation reaction proceeds more rapidly and with greater conversion of the starting material as more acid is employed. A strong acid catalyst is preferably employed in a quantity of from 1 to 5 times more preferably 2 to 3 times the weight of the phenyl substituted alcohol.

The reaction medium is preferably maintained at a temperature of from 50° to 150°C more preferably from 60° to 100° say 60° to 80°C. When using higher temperatures within these ranges e.g. 100° to 150°C particular attention should be paid to any possible reaction between the protonic acid and the primary alcohol. At lower temperatures the rate of oligomerisation of the alkylating agent becomes more significant. Because of this tendency it is preferred to terminate the reaction before all the phenyl propanol has been converted e.g. at 50 or 60% conversion. Where oligomeric alkenes are employed as the alkylating agent a higher temperature e.g. 90 to 120°C is preferably employed.

The desired mono-alkyl phenyl substituted alcohol is preferably separated from the reaction product of the alkylation reaction prior to the final oxidation step. This separation may conveniently be effected by fractional distillation. If this fractionation is carried out sufficiently carefully, it is possible where appropriate to obtain a fraction which is rich in the para substituted product and a second fraction which is correspondingly rich in the meta substituted product.

The alkylation product comprises a mixture of the meta and para isomers of the product. The mixtures often comprise at least 3% of the meta isomer.

The final oxidation step may be carried out by using reaction techniques of oxidation or dehydrogenation known *per se* for this type of oxidation or dehydrogenation. We prefer to dehydrogenate the alcohol in the presence of a suitable catalyst such as Raney nickel, Raney copper or most preferably copper chromite. This reaction may be effected by adding the alcohol to a stirred suspension of the catalyst in a suitable non-volatile solvent which is maintained at a temperature in the range 100 to 220°C and at a subatmospheric pressure. The temperature and pressure are selected so as to arrange that the desired aldehyde product is fractionally distilled from the reaction medium as it is formed which arrangement minimises by-product formation. The invention is illustrated by the following Examples:—

Example 1

Preparation of α-methylcinnamaldehyde

Propionaldehyde (87 gms 15 moles) was added at 50°C over a period of 5 hours to a stirred mixture of benzaldehyde (318 gms, 3 moles), methanol (500 mls), water (500 mls) and sodium hydroxide (25 gms). After a further 1 hour stirring at 50°C, the reaction mixture was brought to neutrality with acetic acid and the methanol fractionated out of the reaction. The organic layer was then separated from the aqueous layer and fractionated to recover the excess of benzaldehyde (109 gms, b. pt. 36—40°C/2 mm Hg $-2.63\times10^2$ $Nm^{-2}$) and often the α-methylcinnamaldehyde (167 gms, b. pt. 100—102°C/2 mm).

Example 2

Preparation of 2-methyl-3-phenylpropanol

α-Methylcinnamaldehyde was hydrogenated using copper chromite (2% by weight) as catalyst at a temperature of 150°C and hydrogen under pressure. After the uptake of hydrogen ceased, the mixture was filtered and distilled to obtain the 2-methyl-3-phenylpropanol (b. pt 100°C/1 mm$-1.32\times10^2$ $Nm^{-2}$).

Example 3

Reaction of t-butyl chloride with 2-methyl-3-phenylpropan-1-ol.

A mixture of t-butyl chloride (0.5 moles) and 2-methyl-3-phenylpropanol (0.5 moles) was added over a period of 60 minutes to a stirred mixture of anhydrous ferric chloride (0.5 moles) and dichloromethane (400 mls), maintaining the temperature between 0°C and 5°C. The reaction mixture was then stirred for a further 5.5 hrs. at this temperature after this period, the ferric chloride was washed out of the reaction with water and then with dilute sodium hydroxide solution. Distillation of the organic layer yielded 90 gms of a product (b. pt 100—140°C/2 torr$-1.32\times10^2$ $Nm^{-2}$) which on analysis by gas liquid chromatography, was found to contain 21 gms of starting material, 9 gms. of the meta-alkylated product, 53.5 gms. of the para-alkylated product and 6.5 gms of the disubstituted product.

Example 4

Reaction of isobutylene with 2-methyl-3-phenyl propan-1-ol

Over a period of 12.5 hours, 114 gms of isobutylene was bubbled into a stirred mixture of 285 gms of 2-methyl-3-phenylpropan-1-ol and 750 gms of phosphoric acid (90% w/w). The temperature was maintained at 70°C. After this time, the reaction mixture was diluted with ethylbenzene and the phosphoric acid washed out, initially with water and then with dilute sodium hydroxide solution. Distillation of the organic layer yielded 306 gms of a product (b. pt. 100—140°C/2 torr$-2.63\times10^2$ $Nm^{-2}$) which was found to contain (by gas liquid chromatography) 137 gms of starting material, 149 gms of the para alkylated product and 11 gms. of the meta alkylated product.

Example 5

Preparation of 3-(t-butylphenyl)-2-methylpropanaldehyde

3-(t-Butylphenyl)-2-methylpropan-1-ol (95% p 5% m) was added continuously to a stirred mixture of

copper chromite and liquid paraffin (the mixture containing about 5% by weight of copper chromite) at a temperature of 180°C and a pressure of 2 mms Hg $2.63 \times 10^2$ $Nm^{-2}$). The required aldehyde was slowly fractionated out from the reaction mixture as a mixture of 95% of the para substituted alcohol and 5% of the meta substituted alcohol.

Example 6

Preparation of 3-(t-butylphenyl)-2-methyl-propanol

150 grams of 3-phenyl-2-methyl-propanol and 375 grams of 90% aqueous phosphoric acid were heated to 100°C and 56 grams of di-isobutylene were added with stirring over a period of 45 minutes. The temperature was maintained at 100° for 5.5 hours and thereafter at 110°C for a period of 6 hours. The product was cooled and the organic layer separated washed three times with water and once with dilute KOH. The product was distilled through a 6 inch (15 cm) vigreaux column to give 124 grams of a product which was analysed to GLC and found to be 3-(t-butylphenyl)-2-methyl-propanol (49.1%) (44.8% para isomer and 4.3% meta isomer).

**Claims for the Contracting States AT BE GB IT SE:**

1. A process for the preparation of a 3(alkyl phenyl) propanal having the formula

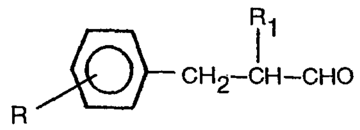

wherein R represents a secondary or tertiary alkyl group having 3 or 4 carbon atoms and $R_1$ represents a hydrogen atom or a methyl group characterised in that the process comprises the steps of:—

(i) reducing an aldehyde which is 3 phenyl prop-2-enal or 3 phenyl 2 methyl prop-2-enal to produce the corresponding 3 phenyl propanol,

(ii) reacting the 3 phenyl propanol with an alkylating agent so as to produce a product comprising a 3-(mono-alkyl phenyl) propanol wherein the alkyl group is a secondary or tertiary alkyl group comprising 3 or 4 carbon atoms and

(iii) oxidising the 3-(mono alkyl phenyl) propanol so as to produce a 3-(monoalkylphenyl) propanal.

2. A process according to claim 1 characterised in that the alkylation agent is selected from the group consisting of alkanols and alkenes having 3 or 4 carbon atoms or oligomers of such alkenes and the alkylation steps is performed in the presence of protonic acid catalyst.

3. A process according to claim 2 characterised in that the reaction is conducted in concentrated phosphoric acid.

4. A process according to claim 2 characterised in that the reaction is conducted in concentrated phosphoric acid present in an amount of from 1 to 5 times the weight of the 3 phenyl propanol.

5. A process according to any of claims 2 to 4 characterised in that the alkylating agent is propylene or isobutylene and the reaction is performed by bubbling the propylene or isobutylene through a liquid medium comprising the propanol and the acid.

6. A process according to any of claims 2 to 4 characterised in that the alkylating agent is tertiary butanol or diisobutylene.

7. A process according to any of claims 2 to 6 characterised in that the alkylation reaction is carried out at a temperature of 50 to 150°C.

8. A process according to claim 2, characterised in that the alkylation reaction is carried out by reacting isobutylene with the 3 phenyl propanol in the presence of from 1 to 5 times the weight of the 3 phenyl propanol of phosphoric acid of at least 85% concentration and at a temperature of from 60 to 100°C.

9. A process according to any preceding claim, characterised in that the oxidation of the 3(monoalkyl phenyl) propanol is carried out by catalytic dehydrogenation.

10. A process according to claim 9, characterised in that the catalyst is selected from the group consisting of Raney nickel, Raney copper and copper chromite.

11. A process according to any preceding claim wherein $R_1$ represents a methyl group.

12. A process for the production of a 3 alkyl phenyl propanol having the formula:—

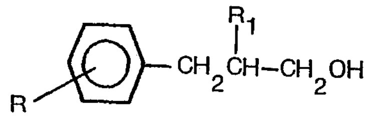

wherein R represents a secondary or tertiary alkyl group having 3 or 4 carbon atoms and $R_1$ represents a hydrogen atom or a methyl group characterised in that the process comprises reacting a 3 phenyl propanol with an alkylating agent, selected from $C_{3-4}$ alkenes, $C_{3-4}$ secondary or tertiary alcohols and oligomers of $C_{3-4}$ alkenes, in the presence of concentrated phosphoric acid.

13. A process according to claim 12 characterised in that the amount of phosphoric acid is 1 to 5 times, by weight, the amount of the propanol.

14. A process according to claim 12 or claim 13 characterised in that the alkylating agent is propylene or isobutylene and the reaction is performed by bubbling the propylene or isobutylene through a liquid medium comprising the propanol and the acid.

15. A process according to any of claims 12 to 14 characterised in that the alkylation is carried out at a temperature of 50 to 150°C.

16. A process according to claim 12 characterised in that it is carried out by reacting isobutylene with 3 phenyl propanol in the presence of from 1 to 5 times the weight of propanol of phosphoric acid of at least 85% concentration and at a temperature of 60 to 100°C.

17. A process according to any of claims 12 to 16 in which the concentration of phosphoric acid is at least 90%.

**Claims for the Contracting States CH DE FR LI NL**

1. A process for the preparation of a 3(alkyl phenyl) propanol having the formula

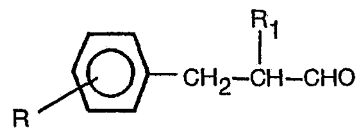

wherein R represents a secondary or tertiary alkyl group having 3 or 4 carbon atoms and $R_1$ represents a hydrogen atom or a methyl group characterised in that the process comprises the steps of:—

(i) reducing an aldehyde which is 3 phenyl prop-2-enal or 3 phenyl 2 methyl prop-2-enal to produce the corresponding 3 phenyl propanol,

(ii) reacting the 3 phenyl propanol in the presence of protonic acid catalyst with an alkylating agent selected from the group consisting of alkanols and alkenes having 3 or 4 carbon atoms or oligomers of such alkenes so as to produce a product comprising a 3-(monoalkylphenyl) propanol wherein the alkyl group is a secondary or tertiary alkyl group comprising 3 or 4 carbon atoms and

(iii) oxidising the 3-(mono alkyl phenyl) propanol so as to produce a 3-(monoalkylphenyl) propanal.

2. A process according to claim 1 characterised in that reaction (ii) is conducted in concentrated phosphoric acid.

3. A process according to claim 1 characterised in that reaction (ii) is conducted in concentrated phosphoric acid present in an amount of from 1 to 5 times the weight of the 3 phenyl propanol.

4. A process according to any preceding claim characterised in that the alkylating agent is propylene or isobutylene and the reaction is performed by bubbling the propylene or isobutylene through a liquid medium comprising the propanol and the acid.

5. A process according to any of claims 1 to 3 characterised in that the alkylating agent is tertiary butanol or diisobutylene.

6. A process according to any preceding claim characterised in that the alkylation reaction is carried out at a temperature of 50 to 150°C.

7. A process according to claim 1, characterised in that the alkylation reaction is carried out by reacting isobutylene with the 3 phenyl propanol in the presence of from 1 to 5 times the weight of the 3 phenyl propanol of phosphoric acid of at least 85% concentration and at a temperature of from 60 to 100°C.

8. A process according to any preceding claim, characterised in that the oxidation of the 3 (monoalkyl phenyl) propanol is carried out by catalytic dehydrogenation.

9. A process according to claim 8, characterised in that the catalyst is selected from the group consisting of Raney nickel, Raney copper and copper chromite.

10. A process according to any preceding claim wherein $R_1$ represents a methyl group.

11. A process for the production of a 3 alkyl phenyl propanol having the formula:—

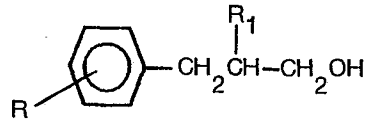

wherein R represents a secondary or tertiary alkyl group having 3 or 4 carbon atoms and $R_1$ represents a hydrogen atom or a methyl group characterised in that the process comprises reacting a 3 phenyl propanol with an alkylating agent, selected from $C_{3-4}$ alkenes, $C_{3-4}$ secondary or tertiary alcohols and oligomers of $C_{3-4}$ alkenes, in the presence of concentrated phosphoric acid.

12. A process according to claim 11 characterised in that the amount of phosphoric acid is 1 to 5 times, by weight, the amount of the propanol.

13. A process according to claim 11 or claim 12 characterised in that the alkylating agent is propylene

or isobutylene and the reaction is performed by bubbling the propylene or isobutylene through a liquid medium comprising the propanol and the acid.

14. A process according to any of claims 11 to 13 characterised in that the alkylation is carried out at a temperature of 50 to 150°C.

15. A process according to claim 11 characterised in that it is carried out by reacting isobutylene with 3 phenyl propanol in the presence of from 1 to 5 times the weight of propanol of phosphoric acid of at least 85% concentration and at a temperature of 60 to 100°C.

16. A process according to any of claims 11 to 15 in which the concentration of phosphoric acid is at least 90%.

**Patentansprüche für die Vertragsstaaten AT BE GB IT SE:**

1. Verfahren zur Herstellung eines 3-(Alkylphenyl)-propanals mit der Formel

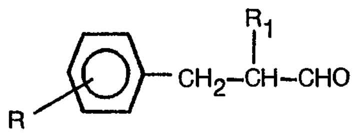

in welcher R für eine sekundäre oder tertiäre Alkylgruppe mit 3 oder 4 Kohlenstoffatomen steht und $R_1$ ein Wasserstoffatom oder eine Methylgruppe bedeutet, dadurch gekennzeichnet, daß man

(i) einen Aldehyd, der 3-Phenyl-prop-2-enal oder 3-Phenyl-2-methyl-prop-2-enal ist, zur Bildung des entsprechenden 3-Phenylpropanols reduziert,

(ii) das 3-Phenylpropanol mit einem Alkylierungsmittel umsetzt, um ein 3-(Monoalkylphenyl)-propanol enthaltendes Produkt herzustellen, in welchem die Alkylgruppe eine sekundäre oder tertiäre Alkylgruppe mit 3 oder 4 Kohlenstoffatomen ist, und

(iii) das 3-(monoalkylphenyl)-propanol zur Bildung eines 3-(Monoalkylphenyl)-propanals oxidiert.

2. Verfahren nach Anspruch 1, dadurch gekennzeichnet, daß das Alkylierungsmittel ausgewählt ist aus der aus Alkanolen und Alkenen mit 3 oder 4 Kohlenstoffatomen oder Oligomeren dieser Alkene bestehenden Gruppe und die Alkylierung in Anwesenheit eines protonischen Säurekatalysators durchgeführt wird.

3. Verfahren nach Anspruch 2, dadurch gekennzeichnet, daß die Reaktion in konz. Phosphorsäure durchgeführt wird.

4. Verfahren nach Anspruch 2, dadurch gekennzeichnet, daß die Reaktion in konz. Phosphorsäure durchgeführt wird, die in einer Menge des 1-bis 5-Fachen des Gewichts des 3-Phenylpropanols anwesend ist.

5. Verfahren nach einem der Ansprüche 2 bis 4, dadurch gekennzeichnet, daß das Alkylierungsmittel Propylen oder Isobutylen ist und die Reaktion erfolgt, indem man das Propylen oder Isobutylen durch ein flüssiges, das Propanol und die Säure enthaltendes Medium hindurchleitet.

6. Verfahren nach einem der Ansprüche 2 bis 4, dadurch gekennzeichnet, daß das Alkylierungsmittel tertiäres Butanol oder Diisobutylen ist.

7. Verfahren nach einem der Ansprüche 2 bis 6, dadurch gekennzeichnet, daß die Alkylierungsreaktion bei einer Temperatur von 50 bis 150°C durchgeführt wird.

8. Verfahren nach Anspruch 2, dadurch gekennzeichnet, daß die Alkylierungsreaktion durchgeführt wird, indem man Isobutylen mit dem 3-Phenylpropanol in Anwesenheit des 1- bis 5- Fachen des Gewichtes des 3-Phenylpropanols an Phosphorsäure einer Konzentration von mindestens 85% und bei einer Temperatur von 60 bis 100°C umsetzt.

9. Verfahren nach einem der vorhergehenden Ansprüche, dadurch gekennzeichnet, daß die Oxidation des 3-(Monoalkylphenyl)-propanols durch katalytische Dehydrierung durchgeführt wird.

10. Verfahren nach Anspruch 9, dadurch gekennzeichnet, daß der Katalysator ausgewählt ist aus der aus Raney-Nickel, Raney-Kupfer und Kupferchromit bestehenden Gruppe.

11. Verfahren nach einem der vorhergehenden Ansprüche, in welche $R_1$ eine Methylgruppe bedeutet.

12. Verfahren zur Herstellung eines 3-Alkylphenylpropanols der Formel

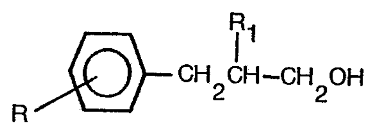

in welcher R für eine sekundäre oder tertiäre Alkylgruppe mit 3 oder 4 Kohlenstoffatomen steht und $R_1$ ein Wasserstoffatom oder eine Methylgruppe bedeutet, dadurch gekennzeichnet, daß das Verfahren die Umsetzung eines 3-Phenylpropanols mit einem Alkylierungsmittel, ausgewählt aus $C_{3-4}$ Alkenen, $C_{3-4}$ sekundären, oder tertiären Alkoholen und Oligomeren der $C_{3-4}$ Alkene, in Anwesenheit von konz. Phosphorsäure umfaßt.

13. Verfahren nach Anspruch 12, dadurch gekennzeichnet, daß die Menge an Phosphorsäure das 1- bis 5-Fache der Gewichtsmenge des Propanols beträgt.

14. Verfahren nach Anspruch 12 oder 13, dadurch gekennzeichnet, daß das Alkylierungsmittel Propylen oder Isobutylen ist und die Reaktion durchgeführt wird, indem man das Propylen oder Isobutylen durch ein flüssiges, das Propanol und die Säure umfassendes Medium hindurchleitet.

15. Verfahren nach einem der Ansprüche 12 bis 14, dadurch gekennzeichnet, daß die Alkylierung bei einer Temperatur von 50 bis 150°C durchgeführt wird.

16. Verfahren nach Anspruch 12, dadurch gekennzeichnet, daß es durchgeführt wird, indem man Isobutylen mit 3-Phenylpropanol in Anwesenheit des 1- bis 5-Fachen des Gewichts des Propanols an Phosphorsäure einer Konzentration von mindestens 85% und bei einer Temperatur von 60 bis 100°C ungesetzt wird.

17. Verfahren nach einem der Ansprüche 12 bis 16, in welchem die Konzentration der Phosphorsäure mindestens 90% ist.

**Patentansprüche für die Vertragsstaaten CH DE FR LI NL;**

1. Verfahren zur Herstellung eines 3-(Alkylphenyl)-propanals mit der Formel

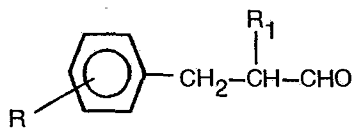

in welcher R für eine sekundäre oder tertiäre Alkylgruppe mit 3 oder 4 Kohlenstoffatomen steht und $R_1$ ein Wasserstoffatom oder eine Methylgruppe bedeutet, dadurch gekennzeichnet, daß man

(i) einen Aldehyd, der 3-Phenyl-prop-2-enal oder 3-Phenyl-2-methyl-prop-2-enal ist, zur Bildung des entsprechenden 3-Phenylpropanols reduziert,

(ii) das 3-Phenylpropanol in Anwesenheit eines protonischen Säurekatalysators mit einem Alkylierungsmittel, ausgewählt aus der aus Alkanolen und Alkenen mit 3 oder 4 Kohlenstoffatomen oder Oligomeren dieser Alkene bestehenden Gruppe, zur Bildung eines 3-(Monoalkylphenyl)-propanol enthaltendes Produktes, in welchem die Alkylgruppe eine sekundäre oder tertiäre Alkylgruppe mit 3 oder 4 Kohlenstoffatomen ist, umsetzt, und

(iii) das 3-(Monoalkylphenyl)-propanol zur Bildung eines 3-(Monoalkylphenyl)-propanals oxidiert.

2. Verfahren nach Anspruch 1, dadurch gekennzeichnet, daß Reaktion (ii) in konz. Phosphorsäure durchgeführt wird.

3. Verfahren nach Anspruch 1, dadurch gekennzeichnet, daß die Reaktion (ii) in konz. Phosphorsäure durchgeführt wird, die in einer Menge des 1- bis 5-Fachen des Gewichts des 3-Phenylpropanols anwesend ist.

4. Verfahren nach einem der vorhergehenden Ansprüche, dadurch gekennzeichnet, daß das Alkylierungsmittel Propylen oder Isobutylen ist und die Reaktion erfolgt, indem man das Propylen oder Isobutylen durch ein flüssiges, das Propanol und die Säure enthaltendes Medium hindurchleitet.

5. Verfahren nach einem der Ansprüche 1 bis 3, dadurch gekennzeichnet, daß das Alkylierungsmittel tertiäres Butanol oder Diisobutylen ist.

6. Verfahren nach einem der vorhergehenden Ansprüche, dadurch gekennzeichnet, daß die Alkylierungsreaktion bei einer Temperatur von 50 bis 150°C durchgeführt wird.

7. Verfahren nach Anspruch 1, dadurch gekennzeichnet, daß die Alkylierungsreaktion durchgeführt wird, indem man Isobutylen mit dem 3-Phenylpropanol in Anwesenheit des 1- bis 5-Fachen des Gewichtes des 3-Phenylpropanols an Phosphorsäure einer Konzentration von mindestens 85% und bei einer Temperatur von 60 bis 100°C umsetzt.

8. Verfahren nach einem der vorhergehenden Ansprüche, dadurch gekennzeichnet, daß die Oxidation des 3-(Monoalkylphenyl)-propanols durch katalytische Dehydrierung durchgeführt wird.

9. Verfahren nach Anspruch 8, dadurch gekennzeichnet, daß der Katalysator ausgewählt ist aus der aus Raney-Nickel, Raney-Kupfer und Kupferchromit bestehenden Gruppe.

10. Verfahren nach einem der vorhergehenden Ansprüche, in welchem $R_1$ eine Methylgruppe bedeutet.

11. Verfahren zur Herstellung eines 3-Alkylphenylpropanols der Formel

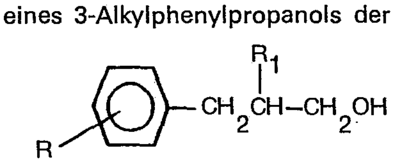

in welcher R für eine sekundäre oder tertiäre Alkylgruppe mit 3 oder 4 Kohlenstoffatomen steht und $R_1$ ein Wasserstoffatom oder eine Methylgruppe bedeutet, dadurch gekennzeichnet, daß das Verfahren die

Umsetzung eines 3-Phenylpropanols mit einem Alkylierungsmittel, ausgewählt aus $C_{3-4}$ Alkenen, $C_{3-4}$ sekundären oder tertiären Alkoholen und Oligomeren der $C_{3-4}$ Alkene, in Anwesenheit von konz. Phosphorsäure umfaßt.

12. Verfahren nach Anspruch 11, dadurch gekennzeichnet, daß die Menge an Phosphorsäure das 1- bis 5-Fache der Gewichtsmenge des Propanols beträgt.

13. Verfahren nach Anspruch 11 oder 12, dadurch gekennzeichnet, daß das Alkylierungsmittel Propylen oder Isobutylen ist und die Reaktion durchgeführt wird, indem man das Propylen oder Isobutylen durch ein flüssiges, das Propanol und die Säure umfassendes Medium hindurchleitet.

14. Verfahren nach einem der Ansprüche 11 bis 13, dadurch gekennzeichnet, daß die Alkylierung bei einer Temperatur von 50 bis 150°C durchgeführt wird.

15. Verfahren nach Anspruch 11, dadurch gekennzeichnet, daß es durchgeführt wird, indem man Isobutylen mit 3-Phenylpropanol in Anwesenheit des 1- bis 5-Fachen des Gewichts des Propanols an Phosphorsäure einer Konzentration von mindestens 85% und bei einer Temperatur von 60 bis 100°C umgesetzt wird.

16. Verfahren nach einem der Ansprüche 11 bis 15, in welchem die Konzentration der Phosphorsäure mindestens 90% ist.

**Revendications pour les Etats Contractants AT BE GB IT SE:**

1. Procédé de préparation d'un (alkylphényl)-3 propanal de formule:

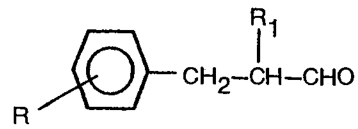

dans laquelle R représente un groupe alkyle secondaire ou tertiaire ayant 3 ou 4 atomes de carbone, et $R_1$ représente un atome d'hydrogène ou un groupe méthyle, caractérisé en ce que le procédé comprend les étapes consistant à:

(i) réduire un aldéhyde, qui est le phényl-3 propène-2 al ou le phényl-3 méthyl-2 propène-2 al, pour produire le phényl-3 propanol correspondant,

(ii) faire réagir le phényl-3 propanol avec un agent d'alkylation de manière à obtenir un produit comprenant un monoalkylphényl)-3 propanol dans lequel le groupe alkyle est un groupe alkyle secondaire ou tertiaire comprenant 3 ou 4 atomes de carbone, et

(iii) oxyder le (monoalkylphényl)-3 propanol afin de produire un (monoalkylphényl)-3 propanal.

2. Procédé selon la revendication 1, caractérisé en ce qu'on choisit l'agent d'alkylation dans l'ensemble constitué par des alcanols et alcènes ayant 3 ou 4 atomes de carbone ou des oligomères de tels alcènes, et l'on effectue l'étape d'alkylation en présence d'un catalyseur acide protonique.

3. Procédé selon la revendication 2, caractérisé en ce qu'on conduit la réaction dans de l'acide phosphorique concentré.

4. Procédé selon la revendication 2, caractérisé en ce qu'on conduit la réaction dans de l'acide phosphorique concentré présent en une quantité de 1 à 5 fois le poids du phényl-3 propanol.

5. Procédé selon l'une quelconque des revendications 2 à 4, caractérisé en ce que l'agent d'alkylation est le propylène ou l'isobutylène, et en ce qu'on effectue la réaction en faisant barboter le propylène ou l'isobutylène dans un milieu liquide comprenant le propanol et l'acide.

6. Procédé selon l'une quelconque des revendications 2 à 4, caractérisé en ce que l'agent d'alkylation est du tertiobutanol ou du diisobutylène.

7. Procédé selon l'une quelconque des revendications 2 à 6, caractérisé en ce qu'on effectue la réaction d'alkylation à une température de 50 à 150°C.

8. Procédé selon la revendication 2, caractérisé en ce qu'on effectue la réaction d'alkylation en faisant réagir l'isobutylène avec le phényl-3 propanol en présence de 1 à 5 fois pe poids du phényl-3 propanol en acide phosphorique à la concentration d'au moins 85% et à une température de 60 à 100°C.

9. Procédé selon l'une quelconque des revendications précédentes, caractérisé en ce qu'on effectue l'oxydation du (monoalkylphényl)-3 propanol par déshydrogénation catalytique.

10. Procédé selon la revendication 9, caractérisé en ce qu'on choisit le catalyseur dans l'ensemble constitué par le nickel de Raney, le cuivre de Raney et le chromite de cuivre.

11. Procédé selon l'une quelconque des revendications précédentes, dans lequel $R_1$ représente un groupe méthyle.

12. Procédé de production d'un alkylphényl-3 propanol de formule:

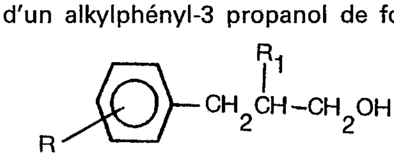

dans laquelle R représente un groupe alkyle secondaire ou tertiaire ayant 3 ou 4 atomes de carbone, et $R_1$ représente un atome d'hydrogène ou un groupe méthyle, caractérisé en ce que le procédé comprend la réaction d'un phényl-3 propanol avec un agent d'alkylation choisi parmi des alcènes en $C_3$ ou $C_4$, des alcools secondaires ou tertiaires en $C_3$ ou $C_4$ et des oligomères d'alcènes en $C_3$ ou $C_4$, en présence d'acide phosphorique concentré.

13. Procédé selon la revendication 12, caractérisé en ce que la quantité de l'acide phosphorique représente 1 à 5 fois, en poids, la quantité de propanol.

14. Procédé selon la revendication 12 ou la revendication 13, caractérisé en ce que l'agent d'alkylation est le propylène ou l'isobutylène, et en ce qu'on effectue la réaction en faisant barboter le propylène ou l'isobutylène dans un mélange liquide comprenant le propanol et l'acide.

15. Procédé selon l'une quelconque des revendications 12 à 14, caractérisé en ce qu'on effectue l'alkylation à une température de 50 à 150°C.

16. Procédé selon la revendication 12, caractérisé en ce qu'on le met en oeuvre en faisant réagir l'isobutylène avec le phényl-3 propanol en présence de 1 à 5 fois le poids de propanol en acide phosphorique, à une concentration d'au moins 85%, et à une température de 60 à 100°C.

17. Procédé selon l'une quelconque des revendications 12 à 16, dans lequel la concentration de l'acide phosphorique est d'au moins 90%.

**Revendications pour les Etats Contractants CH DE FR LI NL;**

1. Procédé de préparation d'un (alkylphényl)-3 propanal de formule:

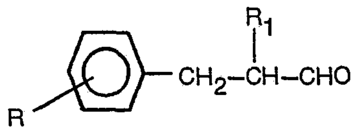

dans laquelle R représente un groupe alkyle secondaire ou tertiaire ayant 3 ou 4 atomes de carbone, et $R_1$ représente un atome d'hydrogène ou un groupe méthyle, caractérisé en ce que le procédé comprend les étapes consistant à:

(i) réduire un aldéhyde, qui est le phényl-3 propène-2 al ou le phényl-3 méthyl-2 propène-2 al, pour produire le phényl-3 propanol correspondant,

(ii) faire réagir le phényl-3 propanol en présence d'un acide protonique comme catalyseur avec un agent d'alkylation choisi parmi les alcanols et les alcènes ayant 3 ou 4 atomes de carbone ou les oligomères de ces alcènes, de manière à obtenir un produit comprenant un (monoalkylphényl)-3 propanol dans lequel le groupe alkyle est un groupe alkyle secondaire ou tertiaire comprenant 3 ou 4 atomes de carbone, et

(iii) oxyder le (monoalkylphényl)-3 propanol afin de produire un (monoalkylphényl)-3 propanal.

2. Procédé selon la revendication 1, caractérisé en ce qu'on conduit la réaction (ii) dans de l'acide phosphorique concentré.

3. Procédé selon la revendication 1, caractérisé en ce qu'on conduit la réaction (ii) dans de l'acide phosphorique concentré, présent en une quantité représentant 1 à 5 fois le poids du phényl-3 propanol.

4. Procédé selon l'une quelconque des revendications précédentes, caractérisé en ce que l'agent d'alkylation est le propylène ou l'isobutyléne, et en ce qu'on effectue la réaction en faisant barboter le propylène ou l'isobutylène dans un milieu liquide comprenant le propanol et l'acide.

5. Procédé selon l'une quelconque des revendications 1 à 3, caractérisé en ce que l'agent d'alkylation est du tertiobutanol ou du diisobutylène.

6. Procédé selon l'une quelconque des revendications précédentes, caractérisé en ce qu'on effectue la réaction d'alkylation à une température de 50 à 150°C.

7. Procédé selon la revendication 1, caractérisé en ce qu'on effectue la réaction d'alkylation en faisant réagir l'isobutylène avec le phényl-3 propanol en présence de 1 à 5 fois le poids du phényl-3 propanol en acide phosphorique à une concentration d'au moins 85% et à une température de 60 à 100°C.

8. Procédé selon l'une quelconque des revendications précédentes, caractérisé en ce que l'on effectue l'oxydation du (monoalkylphényl)-3 propanol par déshydrogénation catalytique.

9. Procédé selon la revendication 8, caractérisé en ce qu'on choisit le catalyseur dans l'ensemble constitué par du nickel de Raney, du cuivre de Raney et du chromite de cuivre.

10. Procédé selon l'une quelconque des revendications précédentes, dans lequel $R_1$ représente un groupe méthyle.

11. Procédé de production d'un (alkylphényl)-3 propanol de formule:

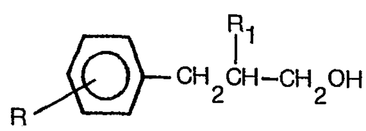

dans laquelle R représente un groupe alkyle secondaire ou tertiaire ayant 3 ou 4 atomes de carbone, et $R_1$ représente un atome d'hydrogène ou un groupe méthyle, caractérisé en ce que le procédé comprend la réaction d'un phényl-3 propanol avec un agent d'alkylation, choisi parmi des alcènes en $C_3$ ou $C_4$, des alcools secondaires ou tertiaires en $C_3$ ou $C_4$ et des oligomères d'alcènes en $C_3$ ou $C_4$, en présence d'acide phosphorique concentré.

12. Procédé selon la revendication 11, caractérisé en ce que la quantité de l'acide phosphorique représente 1 à 5 fois, en poids, la quantité du propanol.

13. Procédé selon la revendication 11 ou la revendication 12, caractérisé en ce que l'agent d'alkylation est le propylène ou l'isobutylène, et en ce qu'on effectue la réaction en faisant barboter le propylène ou l'isobutylène dans un milieu liquide comprenant le propanol et l'acide.

14. Procédé selon l'une quelconque des revendications 11 à 13, caractérisé en ce qu'on effectue l'alkylation à une température de 50 à 150°C.

15. Procédé selon la revendication 11, caractérisé en ce qu'on l'effectue en faisant réagir l'isobutylène avec le phényl-3 propanol en présence de 1 à 5 fois le poids de propanol en acide phosphorique, à une concentration d'au moins 85% et à une température de 60 à 100°C.

16. Procédé selon l'une quelconque des revendications 11 à 15, dans lequel la concentration de l'acide phosphorique est d'au moins 90%.